# EUROPÄISCHE PATENTSCHRIFT

(11) **EP 3 295 975 B1**
(45) Veröffentlichungstag und Bekanntmachung des Hinweises auf die Patenterteilung: **15.05.2019**
(21) Anmeldenummer: 17187517.2
(22) Anmeldetag: 23.08.2017
(51) Int. Cl.: A61M 1/16, B65B 55/02

(54) **STERILGEBINDE MIT EINER VON EINER PRIMÄRVERPACKUNG SEPARATEN STERILBARRIERE SOWIE VERFAHREN ZUR HERSTELLUNG EINES STERILGEBINDES**
STERILE CONTAINER WITH A STERILE BARRIER SEPARATE FROM THE PRIMARY PACKAGING AND METHOD FOR PRODUCING A STERILE CONTAINER
EMBALLAGES ET RÉCIPIENTS STÉRILES DOTÉS D'UNE BARRIÈRE STÉRILE SÉPARÉE D'UN CONDITIONNEMENT PRIMAIRE ET PROCÉDÉ DE FABRICATION DES EMBALLAGES ET RÉCIPIENTS STÉRILES

(30) Priorität: 19.09.2016 DE 102016117599
(43) Veröffentlichungstag der Anmeldung: 21.03.2018
(73) Patentinhaber: B. Braun Avitum AG, 34212 Melsungen (DE)
(72) Erfinder: BURKERT, Sina, Dr., 01157 Dresden (DE)
(74) Vertreter: Winter, Brandl, Fürniss, Hübner, Röss, Kaiser, Polte - Partnerschaft mbB

(56) Entgegenhaltungen:
- US-A1- 2005 063 859
- US-A1- 2010 193 387
- US-A1- 2012 318 727
- US-A1- 2015 359 958

## Beschreibung

Die Erfindung betrifft ein Sterilgebinde mit zumindest einem Hohlfaserfiltermodul, insbesondere einem Dialysator, welches in einem hermetisch dichten Aufnahmevolumen einer Primärverpackung aufgenommen ist und einen von einer Bluteinlassöffnung und einer Blutauslassöffnung, die beispielsweise als Luer-Anschluss ausgestaltet sind, begrenzten Blutraum aufweist. Sie betrifft des Weiteren ein Verfahren zum Erzeugen oder Herstellen eines Sterilgebindes, insbesondere eines solchen erfindungsgemäßen Sterilgebindes nach der vorliegenden Beschreibung oder nach einem der angehängten Ansprüche.

Anforderungen an eine Verpackung für ein Medizinprodukt sind in zahlreichen Normen definiert. Insbesondere eine Verpackung für sterile Medizinprodukte unterliegt strengen Reglementierungen. Im Falle von Hohlfaserfiltermodulen, zum Beispiel Dialysatoren, ist die Norm EN ISO 8637:2014 "Kardiovaskuläre Implantate und extrakorporale Systeme - Hämodialysatoren, Hämodiafilter, Hämofilter und Hämokonzentratoren" besonders wichtig und zu beachten. Im Hinblick auf die Sterilität verpackter Hohlfaserfiltermodule definiert diese Norm, dass blutführende Teile des Geräts steril und folglich geeignet verpackt sein müssen. Bezogen auf ein Hohlfaserfiltermodul, wie einen Dialysator, bedeutet das, dass der Blutraum des Moduls steril sein muss, während der Dialysierflüssigkeitsraum weniger strengen Anforderungen unterliegt.

Zur Sterilisation verpackter Hohlfaserfiltermodule ist die Anwendung von GammaStrahlen im Rahmen einer Bestrahlung allgemein bekannt. Eine Gamma-Strahlen-Sterilisation erfolgt in Abwesenheit von Sauerstoff, um eine oxydative Materialbelastung sowohl des verpackten Hohlfaserfiltermoduls, als auch der dazu genutzten Verpackung bei der Sterilisation zu vermeiden. Bei bekannten Sterilgebinden für Hohlfaserfiltermodule ist jeweils ein Modul zusammen mit einem Sauerstoffabsorber in einer gegenüber der Umgebung hermetisch dichten Primärverpackung verpackt. Der Sauerstoffabsorber wird in die Primärverpackung (Spezialverpackung bestehend aus Polyamid und Polyethylen) gegeben, wodurch Sauerstoff aus dem Medizinprodukt und dem Innenraum der Verpackung annährend vollständig entfernt wird. Die Primärverpackung bildet um den Sauerstoffabsorber und das verpackte Hohlfaserfiltermodul eine hermetisch dichte Hülle, die ein Aufnahmevolumen umgibt und definiert, in dem infolge der Sauerstoffabsorption annährend kein Sauerstoff mehr vorliegt. In diesem Zustand erfolgt die Sterilisation mittels Gamma-Strahlung. Eine Mehrzahl derartig primärverpackter und sterilisierter Einheiten, beispielsweise eine Losgröße vom 20, ist dann in einer Sekundärverpackung aufgenommen. Man kann sagen, dass die Sekundärverpackung dann 20 dicht verpackte und sterile Hohlfaserfiltermodule (Dialysatoren) enthält. Die Primärverpackung definiert dabei das bei der Sterilisation zu sterilisierende Volumen und bildet somit eine Sterilbarriere, die die Barriere zwischen einem sterilen und einem nicht-sterilen Raum darstellt, aus.

Das vorstehend beschriebene Konzept nach dem Stand der Technik birgt große Herausforderungen: So ist einerseits sicherzustellen, dass vor und während der Sterilisation in dem zu sterilisierenden Volumen Sauerstofffreiheit vorliegt. Andererseits ist sicherzustellen, dass nach dem Sterilverpacken, während eines Transports sowie während der gesamten Lagerung (in der Regel bis zu 3 Jahre) der Gebinde bis zum letztlichen Gebrauch, die Sterilität des Hohlfaserfiltermoduls gewahrt ist. Bei den vorstehend beschriebenen bekannten Sterilgebinden ist es von Nachteil, dass durch unsachgemäßen Transport und/oder Lagerung die sensible Primärverpackung minimal perforiert werden kann, zum Beispiel wenn die Primärverpackungen mit den darin aufgenommenen Filtermodulen in einer Sekundärverpackung aneinander reiben. Eine Perforation der Primärverpackung vor der Sterilisation kann dazu führen, dass Sauerstoff von außen in die Verpackung diffundiert und während der Sterilisation vorhanden ist. Dadurch kann es zu Materialbelastungen durch Oxidation kommen. Bei einer Perforation nach der Sterilisation kann es zu einer Unsterilität innerhalb des Aufnahmevolumens der Primärverpackung und damit des Dialysierflüssigkeitsraums des Filtermoduls kommen. Ein weiterer Nachteil bekannter Verpackungskonzepte ist, dass zu jedem einzelnen in einer Primärverpackung verpackten Filtermodul stets ein zusätzlicher Sauerstoffabsorber hinzugefügt werden muss.

Es sind andere Verpackungskonzepte bekannt, zum Beispiel mittels sich an die Gestalt eines Filtermoduls anpassenden Tiefziehverpackungen aus fester Spezialfolie oder unter Verwendung alternativer am Markt zugänglicher Folien. Eine weitere bekannte Lösung, bei der eine Sterilisation ebenfalls mittels Bestrahlung mit GammaStrahlen erfolgt, verwendet eine dichte, mit Aluminium beschichtete Verpackung. Nachteilig bei diesen Konzepten ist ein hoher Kosten- und Produktionsaufwand, Dokumente US2012/318727 A1, US2005/063859 A1, US2010/193387 A1 und US2015/359958 A1 offenbaren Sterilgebinde und Verfahren zum Erzeugen eines Sterilgebindes gemäß dem Stand der Technik.

Ausgehend von dem vorstehend beschriebenen Stand der Technik liegt der vorliegenden Erfindung die Aufgabe zugrunde, die zuvor angeführten Nachteile zu beseitigen, insbesondere ein technisches Konzept bereitzustellen, mit dem das Risiko einer Perforation der Sterilbarriere durch unsachgemäßen Transport und Lagerung reduziert werden kann und somit die Sterilität des Produkts auch im Falle eines unsachgemäßen Transports und/oder einer unsachgemäßen Lagerung abgesichert werden kann.

Nach der Erfindung wird diese Aufgabe gelöst durch ein Sterilgebinde gemäß Anspruch 1 und ein Verfahren zum Erzeugen eines Sterilgebindes gemäß Anspruch 10. Das Sterilgebinde umfasst zumindest ein Hohlfaserfiltermodul, wie einen Dialysator, welches in einem hermetisch dichten Aufnahmevolumen einer Primärverpackung aufgenommen ist und einen von einer Bluteinlassöffnung und einer Blutauslassöffnung begrenzten Blutraum aufweist. Die Erfindung zeichnet sich dadurch aus, dass in der Primärverpackung eine Mehrzahl von Hohlfaserfiltermodulen angeordnet sind und die Sterilbarriere des Blutraumes jedes Hohlfaserfiltermoduls innerhalb der Primärverpackung durch Kappen, wie Schutzkappen oder Protection Caps, bewerkstelligt wird, welche die Bluteinlassöffnung und die Blutauslassöffnung des jeweiligen Hohlfaserfiltermoduls verschließen. Im Hinblick auf ein Verfahren wird die Aufgabe gelöst durch ein Verfahren zum Erzeugen eines Sterilgebindes, insbesondere nach einem der angehängten Ansprüche oder nach der vorliegenden Beschreibung, wobei eine Mehrzahl von Hohlfaserfiltermodulen, insbesondere Dialysatoren, in einer Primärverpackung angeordnet werden, die Primärverpackung nachfolgend ein hermetisch dichtes Aufnahmevolumen ausbildendend verschlossen wird, in dem verschlossenen Aufnahmevolumen vorliegender Sauerstoff mittels eines Sauerstoffabsorbers absorbiert wird, nachfolgend eine Sterilisation mittels Gammastrahlung erfolgt und die Primärverpackung mit den darin verpackten Hohlfaserfiltermodulen in eine Sekundärverpackung eingebracht wird.

Es ist ein besonderer Vorteil der Erfindung, dass die Primärverpackung, die mehrere Hohlfaserfiltermodule / Dialysatoren umfasst, die jeweils einen Blutraum und einen Dialysierflüssigkeitsraum aufweisen, nicht gleichbedeutend mit der Sterilbarriere ist. So wird die Sterilbarriere erfindungsgemäß ausschließlich von den Schutzkappen, die die beiden Blutanschlüsse abdecken, also die Bluteinlass- und die Blutauslassöffnung, die beide vorzugsweise als Luer-Anschlüsse ausgebildet sind, ausgestaltet. Diese Schutzkappen sind robust gegenüber Perforationen, was sich begünstigend auf die Sterilität des Gebindes über dessen Lebensdauer auswirkt. Weiterhin folgt aus dieser Ausgestaltung, dass der steril zu haltende Raum ein deutlich geringeres Volumen aufweist als zuvor, wodurch die zeitliche sowie die wirtschaftliche Effizienz der Sterilisation gesteigert wird. Dadurch, dass erfindungsgemäß mehrere Dialysatoren von einer einzigen Primärverpackung umgeben sind, die die Dialysatoren hermetisch, also luftdicht, gegenüber der Umgebung abdichtet, folgen weitere Vorteile. Denn wenn in einer einzigen Primärverpackung mehrere Dialysatoren enthalten sind, ist im Wesentlichen kein Abschnitt der Primärverpackung zwischen den Dialysatoren vorhanden, weshalb Beschädigungen, wie Perforationen durch Relativbewegungen, der in dem Sterilgebinde verpackten Dialysatoren zueinander weitgehend vermieden, zumindest gegenüber dem Stand der Technik verringert werden können. Außerdem ist die Oberfläche der erfindungsgemäß ausgebildeten Primärverpackung deutlich kleiner als die Oberflächen mehrerer Primärverpackungen nach dem Stand der Technik für eine gleiche Anzahl an Filtermodulen.

Vorteilhafte Ausführungsformen der Erfindung sind in den Unteransprüchen beansprucht und werden nachfolgend näher erläutert.

So ist es von Vorteil, wenn die Verschlusskappen / Schutzkappen / Kappen kraftschlüssig mit der Bluteinlassöffnung respektive der Blutauslassöffnung verbunden sind. Sobald die entsprechenden Öffnungen als Luer-Öffnungen beziehungsweise Luer-Anschlüsse ausgebildet sind, lässt sich der Kraftschluss leicht und zuverlässig über einen Luer-Lock realisieren. Alternativ zur kraftschlüssigen Verbindung ist vor allem auch ein formschlüssiges Aufsetzen der Verschlusskappen vorteilhaft. Hierbei bietet sich ein Aufclipsen, Aufcrimpen oder ein Aufstecken an.

In einer weiteren vorteilhaften Ausführungsform ist die Primärverpackung als durchstoßsichere Folie ausgestaltet, um ein Einbringen von Perforationen in die Primärverpackung zu verhindern. Eine solche Folie zeichnet sich durch eine hohe Zähigkeit verglichen mit üblichen Folien, wie sie beispielsweise im Stand der Technik für Primärverpackungen eingesetzt werden, aus. Nach einer Ausführungsform der Erfindung ist die Primärverpackung auch durch eine flexible Folie gebildet. Durch ihre Flexibilität kann sie sich gut an die Form der darin verpackten Filtermodule anpassen. Die Folie soll Sauerstofffreiheit zumindest während der Sterilisation sicherstellen. Vorzugsweise ist sie außerdem über einen längeren Zeitraum hermetisch dicht, zum Beispiel über ein bis drei Jahre, sodass auch über einen solchen Zeitraum Sauerstofffreiheit vorliegt. In einem Fall, in dem nach der Sterilisation im Verlauf eines längeren Zeitraums dennoch Sauerstoff durch die Folie migriert, ist das kein Nachteil, solange mittels der Schutzkappen eine Sterilität sichergestellt ist, da Sauerstofffreiheit auf die Qualität eines einmal sterilisierten Produkts keine vermindernde Wirkung ausübt.

Sobald in die Primärverpackung oder in das Aufnahmevolumen ein Sauerstoffabsorber eingebracht ist, um eine oxydative Materialbelastung zu vermeiden, ist die Sterilisation zuverlässig durchführbar, sodass an deren Ende unerwünschte Mikroorganismen entfernt sind. Dadurch, dass erfindungsgemäß eine Primärverpackung mehrere Dialysatoren beinhaltet, besteht ein weiterer Vorteil darin, dass, anstatt für jedes einzelne Filtermodul jeweils einen Sauerstoffabsorber vorsehen zu müssen, nur ein einziger Sauerstoffabsorber für die Mehrzahl an Filtermodulen verwendet werden muss. Das vereinfacht Handhabungs- und Fertigungsvorgänge bei der Herstellung des Sterilgebindes.

Alternativ oder zusätzlich kann der Sauerstoffabsorber in die durchstoßsichere Folie integriert sein, die Primärverpackung also durch eine sauerstoffabsorbierende Folie gebildet sein. Insbesondere kann eine bereits in der Nahrungsmittelindustrie verwendete sauerstoffabsorbierende Folie verwendet werden. Durch die Verwendung von geeignet großen Sauerstoffabsorbern bzw. einer Folie mit ausreichender Kapazität kann eine dichteste Packung erzeugt werden, was die Stoßkontakte zwischen den Dialysatoren stark verringert. D.h. der Luftsauerstoff wird entfernt, die Verpackung zieht sich zusammen und drückt die Dialysatoren eng zusammen.

Ein weiterer Vorteil folgt daraus, wenn die Primärverpackung infolge einer Sauerstoffabsorption durch den Sauerstoffabsorber zusammengezogen ist, insbesondere derart zusammengezogen, dass sie an den darin angeordneten Hohlfaserfiltermodulen / Dialysatoren anliegt und diese gegeneinander fixiert. Auf diese Weise wird, ähnlich wie bei einer Verwendung einer Schrumpffolie, ein relativ festes Verpackungsgebinde erstellt, bei dem die in der Primärverpackung verpackten Filtermodule gegeneinander und gegen die Primärverpackung anliegen, so dass jedes Modul im Verpackungsverbund eine relativ stabile Lage besitzt. Gegenseitige Verlagerung der Filtermodule zueinander und zur Primärverpackung können damit in vorteilhafter Weise weitgehend vermieden werden. Damit wird die Wahrscheinlichkeit einer Perforation der Primärverpackung zusätzlich vermindert, während die Perforation der Sterilbarriere weiterhin ausgeschlossen ist. Im Hinblick auf das erfindungsgemäße Verfahren kann man sagen, dass das Aufnahmevolumen der Primärverpackung infolge der Sauerstoffabsorption derart verringert wird, dass die darin aufgenommenen Hohlfaserfiltermodule durch Anlage an der Primärverpackung und/oder durch gegenseitige Anlage aneinander lagestabil fixiert sind.

Eine weitere Ausführungsform der Erfindung ist dadurch gekennzeichnet, dass in dem Aufnahmevolumen zwischen zumindest einem Teil der Hohlfaserfiltermodule eine Zwischenlage, zum Beispiel in Form von Pappe, Folie, Luftpolsterfolie oder Schaumpolster, angeordnet ist. Solche Zwischenlagen können Relativbewegungen der Filtermodule zueinander in vorteilhafter Weise unterbinden, zumindest einschränken. Sie dienen somit nicht nur dem Schutz der verpackten Filtermodule, sondern außerdem dem Schutz der Primärverpackung, indem sie Perforationen infolge von Bewegungen der Filtermodule vermeiden kann. Die Zwischenlagen können planparallel zueinander, oder auch einander kreuzend angeordnet sein.

Nach einer weiteren Ausführungsform kann die Primärverpackung von einer Sekundärverpackung umgeben sein, die als fester Behälter, insbesondere durch einen Karton, ausgebildet ist. Die Verwendung anderer Materialen, wie zum Beispiel Holz oder Kunststoff oder Metall liegt im Rahmen der Erfindung.

Zwischen der Sekundärverpackung und der Primärverpackung kann eine Zwischenlage, zum Beispiel in Form von Pappe, Folie, Luftpolsterfolie oder Schaumpolster, angeordnet sein, um auf die Dialysatoren wirkende Stöße abzudämpfen. Neben Stößen haben mittels jener Zwischenlage auch Erschütterungen oder Vibrationen, die unter Umständen von außen auf das Sterilgebinde wirken, keinen negativen Einfluss auf die Sterilbarriere oder auch die Primärverpackung. Die Anordnung der in der Primärverpackung vorhandenen Dialysatoren kann in der Sekundärverpackung vertikal oder horizontal sein. Das bedeutet, dass die Sekundärverpackung von oben oder von der Seite aus befüllt werden kann.

Im Hinblick auf das erfindungsgemäße Verfahren ist eine Ausführungsform dadurch gekennzeichnet, dass die Sterilisation nach dem Einbringen der Primärverpackung mit den darin verpackten Hohlfaserfiltermodulen / Dialysatoren in die Sekundärverpackung erfolgt. Dadurch wird ein besonders guter Schutz der Primärverpackung während der Sterilisation erzielt.

Man kann auch sagen, dass die Erfindung ein alternatives Verpackungskonzept zur Verfügung stellt, bei dem ein Sauerstoff absorbierendes Material zur Erzeugung einer dichtesten Packung von mehreren Hohlfaserfiltermodulen, insbesondere Dialysatoren, auf dem Weg zur Sterilisation, unter Einhaltung aller regulatorischen Vorschriften für Dialysatoren verwendet wird. In einer ersten Option wird eine Folie mit zusätzlichem Sauerstoffabsorber verwendet. In einer zweiten Option wird eine Spezialfolie mit einem integrierten Sauerstoffabsorber verwendet. Beide Optionen können kombiniert werden. Nach der Erfindung kann für die Primärverpackung insbesondere eine verglichen mit üblichen Verpackungsfolien sehr dicke, durchstoßsichere Folie verwendet werden. Zusätzliche flexible Zwischenlagen können verwendet werden und verringern weiterhin störende Stoßeffekte.

Durch die Erfindung können insbesondere die folgenden Vorteile bzw. Verbesserungen bewirkt werden:
- Vereinfachung der Sterilisierung und deren Aufrechterhaltung aufgrund des kleineren sterilen Raums und aufgrund der robusten Schutzkappen;
- risikominimierende Maßnahme zur Vermeidung von Perforationen in der Primärverpackung;
- risikominimierende Maßnahme zur Vermeidung einer Zerstörung der Sterilbarriere;
- Vermeiden von Risiken für einen Patienten im Falle von unsachgemäßem Transport der Filtermodule;
- kostengünstiger als bekannte Konzepte, da nicht jedes Filtermodul / jeder Dialysator eine Primärverpackung samt Sauerstoffabsorber erhält, sondern jeweils die in der Primärverpackung verpackte Mehrzahl, zum Beispiel 20 Stück.

Die Erfindung wird im Folgenden anhand von beispielhaften, nicht einschränkenden und in den angehängten Figuren gezeigten Ausführungsformen näher erläutert. Dabei zeigt:
Fig. 1 einen Dialysator mit einer Bluteinlassöffnung und einer Blutauslassöffnung, die jeweils mit einer Schutzkappe verschlossen sind.
Fig. 2 eine erste Ausführungsform der Erfindung in einer schematischen Darstellung,
Fig. 3 eine zweite Ausführungsform der Erfindung in einer schematischen Darstellung,
Fig. 4 eine dritte Ausführungsform der Erfindung in einer schematischen Darstellung und
Fig. 5 eine vierte Ausführungsform der Erfindung in einer schematischen Darstellung.

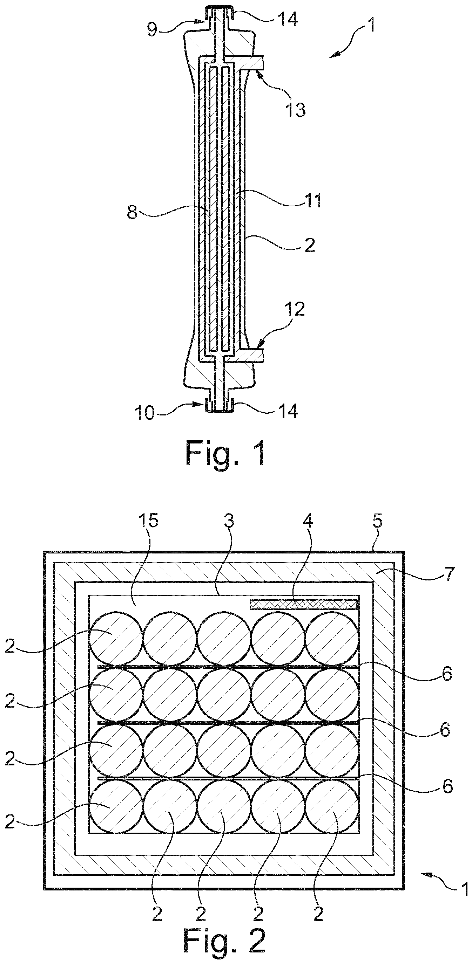

In Fig. 1 ist ein Teil eines erfindungsgemäßen Sterilgebindes 1 dargestellt. Dieser Teil ist aus einem Dialysator 2 und zwei Schutzkappen 14 zusammengesetzt. Die eine Schutzkappe 14 ist auf eine Bluteinlassöffnung 9, die andere Schutzkappe 14 ist auf eine Blutauslassöffnung 10 aufgesetzt. Die Bluteinlass- und die Blutauslassöffnung 9, 10 befinden sich in der Längsrichtung des Dialysators 2 verlaufend jeweils an zwei gegenüberliegenden Enden des Dialysators 2.

Zwischen den Öffnungen 9, 10 ist ein Blutraum 8 definiert. Dieser ist von den Schutzkappen 14 steril abgedichtet. Während ein zu dialysierendes Medium, vorzugsweise Blut, dem Blutraum 8 über die Bluteinlassöffnung 9, die als Luer-Anschluss ausgebildet ist, zugeführt wird, verlässt jenes Medium den Blutraum über die Blutauslassöffnung 10. Im Gegenstromprinzip hierzu wird eine Dialysierflüssigkeit über eine Dialysierflüssigkeitseinlassöffnung 12 einem Dialysierflüssigkeitsraum 11 zugeführt und über eine Dialysierflüssigkeitsauslassöffnung 13 dem Dialysierflüssigkeitsraum 11 entnommen.

Die Blutöffnungen 9, 10 sind in der Längsrichtung des Dialysators 2 verlaufend, während die Dialysierflüssigkeitsöffnungen 12, 13 hierzu abgewinkelt angeordnet sind.

Der Kraftschluss zwischen den Schutzkappen 14 und den Öffnungen 9, 10 ist vorzugsweise über einen Luer-Lock ausgestaltet.

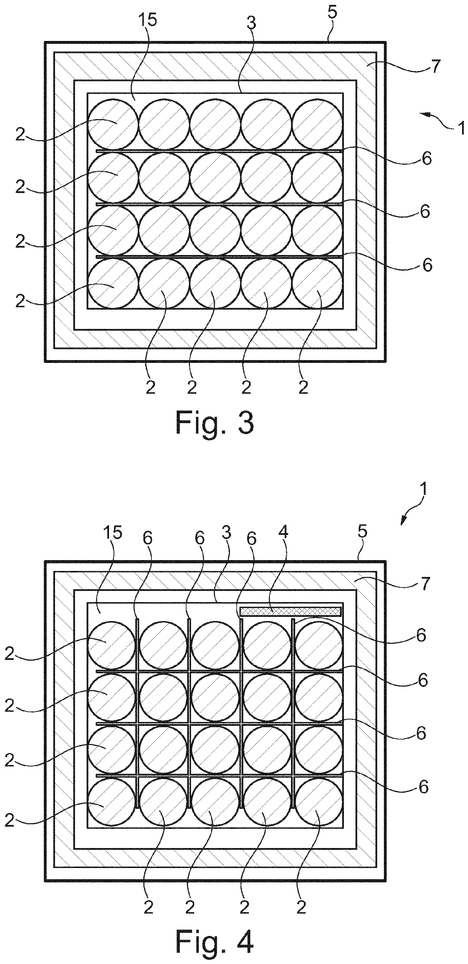

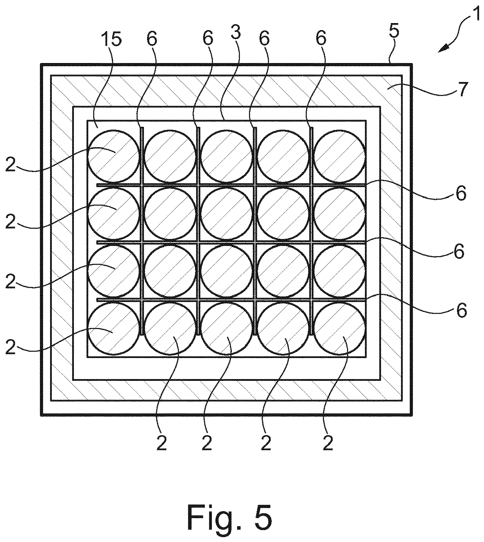

Die Figuren 2 und 3 beschreiben ein Konzept nach der Erfindung unter der Berücksichtigung, dass Kartons 5 (Sekundärverpackungen 5) von oben befüllt werden bzw. sind. Man legt die Dialysatoren 2 sozusagen nebeneinander. In der Ausführungsform der Figuren 4 und 5 werden bzw. sind die Dialysatoren 2, in ein vorgefertigtes, flexibles polsterndes Netz, das eine Zwischenlage 6 ausbildet, (ähnlich einer Weinverpackung) gesteckt, d.h. sie stehen senkrecht. Es entsteht in alle Richtungen eine Grenzschicht zu benachbarten Dialysatoren 2. Zwischen dem zuvor dargestellten Blutraum 8 und dem von der Primärverpackung 3 gebildeten Aufnahmevolumen 15 ist die Sterilbarriere über die Schutzkappen 14 realisiert.

Die Figuren 2 bis 5 zeigen außerdem, dass in einer Primärverpackung 3 eine Mehrzahl an Dialysatoren 2 aufgenommen ist. Im dargestellten Fall enthält eine einzige Primärverpackung 3 zwanzig Dialysatoren 2, die in vier Reihen zu je fünf Dialysatoren 2 angeordnet sind.

Die Primärverpackung 3 der Ausführungsformen der Figuren 2 und 3 sind aus flexiblen Folien gebildet, beispielsweise bestehend aus Polyamid und/oder Polyethylen. In diesen Ausführungsformen ist in jeder Primärverpackung 3 ein Sauerstoffabsorber 4 vorgesehen und angeordnet, also ein einziger Absorber 4 für zwanzig Filtermodule 2. Zwischen den vier Reihen an Dialysatoren 2 ist jeweils die Zwischenlage 6 oder der Zwischenboden 6 angeordnet. Des Weiteren ist zwischen der Primärverpackung 3 und der Sekundärverpackung 5 ein Zwischenpolster 7 in Form einer Luft- oder Schaumpolsterung 7 angeordnet, die eine Übertragung von Stößen, Schlägen und Vibrationen von der Sekundärverpackung 5 auf die Primärverpackung 3 verhindert oder zumindest behindert.

Die Primärverpackungen 3 der Ausführungsformen der Figuren 3 und 5 sind aus flexiblen Folien gebildet, die an sich sauerstoffabsorbierende Eigenschaften aufweisen. Insbesondere handelt es sich bei diesen Folien um bereits in der Nahrungsmittelindustrie verwendete sauerstoffabsorbierende Folien. Zwischen den vier Reihen an Dialysatoren 2 ist ein kreuzartiges oder matrixartiges Netz aus Zwischenlagen 6 oder Zwischenböden 6 angeordnet, derart, dass jeder Kontakt von Dialysatoren 2 untereinander unterbunden wird. Des Weiteren ist zwischen der Primärverpackung 3 und der Sekundärverpackung 5 ein Zwischenpolster 7 in Form einer Luft- oder Schaumpolsterung 7 angeordnet, die eine Übertragung von Stößen, Schlägen und Vibrationen von der Sekundärverpackung 5 auf die Primärverpackung 3 verhindert oder zumindest behindert.

Dadurch, dass erfindungsgemäß die Sterilbarriere von den Schutzkappen 14 realisiert ist, ist ein Sauerstoffabsorber 4 für sämtliche Dialysatoren 2 in einer Sekundärverpackung 5 eingesetzt. Die Anzahl an Dialysatoren 2 in der Sekundärverpackung 5 entspricht erfindungsgemäß der Anzahl an Dialysatoren 2 in der Primärverpackung 3

### Bezugszeichen

- 1: Sterilgebinde
- 2: Dialysator
- 3: Primärverpackung
- 4: Sauerstoffabsorber
- 5: Sekundärverpackung, Karton
- 6: Zwischenlage, Zwischenboden
- 7: Zwischenpolster
- 8: Blutraum
- 9: Bluteinlassöffnung
- 10: Blutauslassöffnung
- 11: Dialysierflüssigkeitsraum
- 12: Dialysierflüssigkeitseinlassöffnung
- 13: Dialysierflüssigkeitsauslassöffnung
- 14: Schutzkappe
- 15: Aufnahmevolumen

## Patentansprüche

1. Sterilgebinde (1) mit zumindest einem Hohlfaserfiltermodul (2), insbesondere Dialysator (2), welches in einem hermetisch dichten Aufnahmevolumen (15) einer Primärverpackung (3) aufgenommen ist und einen von einer Bluteinlassöffnung (9) und einer Blutauslassöffnung (10) begrenzten Blutraum (8) aufweist,
**dadurch gekennzeichnet, dass**
in der Primärverpackung (3) eine Mehrzahl von Hohlfaserfiltermodulen (2) angeordnet sind und die Sterilbarriere des Blutraumes (8) jedes Hohlfaserfiltermoduls (2) innerhalb der Primärverpackung (3) durch Kappen (14) bewerkstelligt wird, welche die Bluteinlassöffnung (9) und die Blutauslassöffnung (10) des jeweiligen Hohlfaserfiltermoduls (2) verschließen.

2. Sterilgebinde (1) nach Anspruch 1, **dadurch gekennzeichnet, dass** die Kappen (14) kraftschlüssig mit der Bluteinlassöffnung (9) und/oder der Blutauslassöffnung (10) verbunden ist.

3. Sterilgebinde (1) nach einem der Ansprüche 1 oder 2, **dadurch gekennzeichnet, dass** die Primärverpackung (3) als durchstoßsichere Folie ausgestaltet ist, um ein Einbringen von Perforationen in die Primärverpackung (3) zu verhindern.

4. Sterilgebinde (1) nach einem der Ansprüche 1 bis 3, **dadurch gekennzeichnet, dass** in die Primärverpackung (3) oder in das Aufnahmevolumen (15) ein Sauerstoffabsorber (4) eingebracht ist, um eine oxydative Materialbelastung zu vermeiden.

5. Sterilgebinde (1) nach Anspruch 4, **dadurch gekennzeichnet, dass** der Sauerstoffabsorber (4) in die durchstoßsichere Folie integriert ist.

6. Sterilgebinde (1) nach einem der Ansprüche 4 oder 5, **dadurch gekennzeichnet, dass** die Primärverpackung (3) infolge einer Sauerstoffabsorption durch den Sauerstoffabsorber (4) zusammengezogen ist und an den darin angeordneten Hohlfaserfiltermodulen (2) anliegt, um diese gegeneinander zu fixieren.

7. Sterilgebinde (1) nach einem der Ansprüche 1 bis 6, **dadurch gekennzeichnet, dass** in dem Aufnahmevolumen (15) zwischen zumindest einem Teil der Hohlfaserfiltermodule (2) eine Zwischenlage (6) angeordnet ist.

8. Sterilgebinde (1) nach einem der Ansprüche 1 bis 7, **dadurch gekennzeichnet, dass** die Primärverpackung (3) von einer Sekundärverpackung (5), die als fester Behälter ausgebildet ist, umgeben ist.

9. Sterilgebinde (1) nach einem der Ansprüche 1 bis 8, **dadurch gekennzeichnet, dass** zwischen der Sekundärverpackung (5) und der Primärverpackung (3) ein Zwischenpolster (7) angeordnet ist, um auf die Hohlfaserfiltermodule (2) wirkende Stöße abzudämpfen.

10. Verfahren zum Erzeugen eines Sterilgebindes (1), wobei
eine Mehrzahl von Hohlfaserfiltermodulen (2) in einer Primärverpackung (3) angeordnet werden,
die Primärverpackung (3) nachfolgend ein hermetisch dichtes Aufnahmevolumen (15) ausbildendend verschlossen wird,
in dem verschlossenen Aufnahmevolumen (15) vorliegender Sauerstoff mittels eines Sauerstoffabsorbers (4) absorbiert wird,
nachfolgend eine Sterilisation mittels Gammastrahlung erfolgt und
die Primärverpackung (3) mit den darin verpackten Hohlfaserfiltermodulen (2) in eine Sekundärverpackung (5) eingebracht wird.

11. Verfahren nach Anspruch 10, **dadurch gekennzeichnet, dass** das Aufnahmevolumen (15) der Primärverpackung (3) infolge der Sauerstoffabsorption derart verringert wird, dass die darin aufgenommenen Hohlfaserfiltermodule (2) durch Anlage an der Primärverpackung (3) und/oder durch gegenseitige Anlage aneinander lagestabil fixiert sind.

## Claims

1. A sterile container (1) comprising at least one hollow fiber filter module (2), especially dialyzer (2), which is accommodated in a hermetically sealed holding volume (15) of a primary package (3) and includes a blood compartment (8) delimited by a blood inlet opening (9) and a blood outlet opening (10),
**characterized in that**
in the primary package (3) a plurality of hollow fiber filter modules (2) is arranged and the sterile barrier of the blood compartment (8) of each hollow fiber filter module (2) inside the primary package (3) is implemented by caps (14) which close the blood inlet opening (9) and the blood outlet opening (10) of the respective hollow fiber filter module (2).

2. The sterile container (1) according to claim 1, **characterized in that** the caps (14) are connected to the blood inlet opening (9) and/or to the blood outlet opening (10) via a frictional connection.

3. The sterile container (1) according to one of claims 1 or 2, **characterized in that** the primary package (3) is in the form of a puncture-proof film so as to prevent perforations from being introduced to the primary package (3).

4. The sterile container (1) according to one of claims 1 to 3, **characterized in that** an oxygen absorber (4) is introduced into the primary package (3) or into the holding volume (15) so as to avoid oxidative material stress.

5. The sterile container (1) according to claim 4, **characterized in that** the oxygen absorber (4) is integrated in the puncture-proof film.

6. The sterile container (1) according to one of claims 4 or 5, **characterized in that** the primary package (3) is contracted due to oxygen absorption by the oxygen absorber (4) and is adjacent to the hollow fiber filter modules (2) disposed therein so as to fix the latter against each other.

7. The sterile container (1) according to according to one of claims 1 to 6, **characterized in that** in the holding volume (15) an intermediate layer (6) is interposed between at least part of the hollow fiber filter modules (2).

8. The sterile container (1) according to one of claims 1 to 7, **characterized in that** the primary package (3) is surrounded by a secondary package (5) which is in the form of a firm container.

9. The sterile container (1) according to one of claims 1 to 8, **characterized in that** between the secondary package (5) and the primary package (3) an intermediate pad (7) is interposed so as to dampen impacts acting on the hollow fiber filter modules (2).

10. A method of producing a sterile container (1), wherein
a plurality of hollow fiber filter modules (2) is arranged in a primary package (3),
the primary package (3) then is closed while forming a hermetically sealed holding volume (15),
oxygen present in the closed holding volume (15) is absorbed by means of an oxygen absorber (4),
subsequently sterilization takes place by means of gamma radiation and
the primary package (3) including the hollow fiber filter modules (2) packaged therein is introduced to a secondary package (5).

11. The method according to claim 10, **characterized in that** the holding volume (15) of the primary package (3) is reduced due to the oxygen absorption so that the hollow fiber filter modules (2) accommodated therein are fixed in a stable position by contacting the primary package (3) and/or by mutual contact.

## Revendications

1. Récipient stérile (1) avec au moins un module de filtre de fibre creuse (2), en particulier un dialyseur (2), qui est reçu dans un volume de réception (15) hermétiquement étanche d'un emballage primaire (3) et présente un espace pour le sang (8) délimité par une ouverture d'entrée de sang (9) et une ouverture de sortie de sang (10),
**caractérisé en ce que**
une pluralité de modules de filtre de fibre creuse (2) sont agencés dans l'emballage primaire (3) et la barrière stérile de l'espace pour le sang (8) de chaque module de filtre de fibre creuse (2) est réalisée à l'intérieur de l'emballage primaire (3) par des couvercles (14) qui ferment l'ouverture d'entrée de sang (9) et l'ouverture de sortie de sang (10) du module de filtre de fibre creuse (2) respectif.

2. Récipient stérile (1) selon la revendication 1, **caractérisé en ce que** les couvercles (14) est raccordé à force à l'ouverture d'entrée de sang (9) et/ou à l'ouverture de sortie de sang (10).

3. Récipient stérile (1) selon l'une quelconque des revendications 1 ou 2, **caractérisé en ce que** l'emballage primaire (3) est configuré en tant que film résistant aux perforations afin d'empêcher une introduction de perforations dans l'emballage primaire (3).

4. Récipient stérile (1) selon l'une quelconque des revendications 1 à 3, **caractérisé en ce qu'**un absorbeur d'oxygène (4) est introduit dans l'emballage primaire (3) ou dans le volume de réception (15) afin d'éviter une sollicitation de matériau d'oxydation.

5. Récipient stérile (1) selon la revendication 4, **caractérisé en ce que** l'absorbeur d'oxygène (4) est intégré dans le film résistant aux perforations.

6. Récipient stérile (1) selon l'une quelconque des revendications 4 ou 5, **caractérisé en ce que** l'emballage primaire (3) est resserré à la suite d'une absorption d'oxygène par l'absorbeur d'oxygène (4) et repose contre les modules de filtre de fibre creuse (2) agencés dans celui-ci afin de fixer ceux-ci l'un contre l'autre.

7. Récipient stérile (1) selon l'une quelconque des revendications 1 à 6, **caractérisé en ce qu'**une couche intermédiaire (6) est agencée dans le volume de réception (15) entre au moins une partie des modules de filtre de fibre creuse (2).

8. Récipient stérile (1) selon l'une quelconque des revendications 1 à 7, **caractérisé en ce que** l'emballage primaire (3) est entouré par un emballage secondaire (5) qui est réalisé en tant que récipient fixe.

9. Récipient stérile (1) selon l'une quelconque des revendications 1 à 8, **caractérisé en ce qu'**un rembourrage intermédiaire (7) est agencé entre l'emballage secondaire (5) et l'emballage primaire (3) afin d'amortir des coups agissant sur les modules de filtre de fibre creuse (2).

10. Procédé de génération d'un récipient stérile (1), dans lequel
une pluralité de modules de filtre de fibre creuse (2) sont agencés dans un emballage primaire (3),
l'emballage primaire (3) est ensuite refermé en réalisant un volume de réception (15) hermétiquement étanche,
de l'oxygène se trouvant dans le volume de réception (15) fermé est absorbé au moyen d'un absorbeur d'oxygène (4),
puis une stérilisation est effectuée au moyen d'un rayonnement gamma et
l'emballage primaire (3) est introduit avec les modules de filtre de fibre creuse (2) emballés dans celui-ci dans un emballage secondaire (5).

11. Procédé selon la revendication 10, **caractérisé en ce que** le volume de réception (15) de l'emballage primaire (3) est réduit à la suite de l'absorption d'oxygène de telle manière que les modules de filtre de fibre creuse (2) reçus dans celui-ci soient fixés de manière stable en position par repos contre l'emballage primaire (3) et/ou par appui mutuel l'un contre l'autre.
